Europäisches Patentamt

European Patent Office

Office européen des brevets

(19)

(11) Veröffentlichungsnummer: **0 158 213**
A2

(12) # EUROPÄISCHE PATENTANMELDUNG

(21) Anmeldenummer: 85103557.6

(22) Anmeldetag: 26.03.85

(51) Int. Cl.⁴: **C 12 M 1/00**

(30) Priorität: 27.03.84 DE 3411264

(43) Veröffentlichungstag der Anmeldung:
16.10.85 Patentblatt 85/42

(84) Benannte Vertragsstaaten:
AT CH DE FR LI NL

(71) Anmelder: Kirmair, Lorenz
Am Griesberg 4 Kirchdorf
D-8206 Bruckmühl(DE)

(72) Erfinder: Kirmair, Lorenz
Am Griesberg 4 Kirchdorf
D-8206 Bruckmühl(DE)

(74) Vertreter: Seeger, Wolfgang, Dipl.-Phys.
European Patent Attorney Bereiteranger 15
D-8000 München 90(DE)

(54) **Verfahren und Anlagen zur Produktion von Biogas.**

(57) Vorrichtung zur Erzeugung von Biogas aus Biomasse, insbesondere aus landwirtschaftlichen Reststoffen wie Gülle und Fäkalien, im Durchgangsverfahren, mit einer Zuführleitung für frische Biomasse, die in den unteren Bereich eines Gärbehälters mündet, der eine Heizvorrichtung umfaßt, dadurch gekennzeichnet, daß die Zuführungsleitung aus wärmeleitendem Material besteht, sich durch die vergärende bzw. vergorene Biomasse in den unteren Behälterraum erstreckt und sich von dort in mindestens zwei Rohrleitungen verzweigt, die in einen Ringbereich des Gärbehälters münden, und daß Heizeinrichtungen zu beiden Seiten auf einem Teil dieses Ringbereichs, die frisch eingeführte Biomasse von zwei Seiten aufwärmend, vorgesehen sind.

Croydon Printing Company Ltd.

0158213

Verfahren und Vorrichtungen
zur Erzeugung von Biogas

Die Erfindung betrifft ein Verfahren zur Erzeugung von Biogas aus organischen Materialien, insbesondere aus landwirtschaftlichen Reststoffen wie Gülle und Fäkalien, bei welchem die Biomasse vergleichsweise langsam in den unteren Bereich eines Gärbehälters eingebracht, aufgeheizt, im Durchgangsverfahren vergoren wird und nach Abgabe von Biogas nach unten abgeleitet wird.

Ein Verfahren der genannten Art ist aus der DE-OS 30 10 183 bekannt. Bei diesem bekannten Verfahren wird frische Biomasse aus einer Sammelgrube, in welcher sie vorgeheizt wird, von unten in einen Gärbehälter unter Druck eingepumpt, der in seiner Außenwand Heizungsrohre umfaßt. Der Gärbehälter geht in einem kegelförmigen Mantel über, von dessen Spitze aus eine Gasleitung zu einem Gasbehälter führt. Relativ dicht unterhalb der Spitze dieses kegelförmigen Daches des Gärbehälters befindet sich die Öffnung eines zentralen Austragrohres, durch welches die Biomasse, die durch den Druck der mittels der Pumpe eingefüllten frischen Biomasse nach oben gedrückt wird, nach unten durch den zentralen Abschnitt des Gärbehälters wieder abläuft.

Dieses bekannte Verfahren und die offenbarte Vorrichtung weisen zum einen den Nachteil auf, daß die Biomasse im Sammelbehälter vorgeheizt werden muß. Darüber hinaus weisen sie den Nachteil auf, daß sie keine

- 1 -

0158213

langsame und gleichmäßige Strömung der Biomasse erlauben, da die frische Biomasse von unten nur an einer Seite durch ein einziges Zuleitungsrohr unter Druck eingeführt wird. Ferner weisen sie auch den Nachteil eines nur geringen Wirkungsgrades auf, da die noch nach oben steigende, also noch nicht voll vergorene oder ausgegaste Biomasse unter dem Druck des in der Kegelspitze befindlichen Gases in das Fallrohr gedrückt wird und nach unten abläuft. Aus diesem Grund sind in der genannten DE-OS 30 10 183 auch in der einzigen Figur eine ganze Reihe von Gärbehältern in Reihe hintereinander geschaltet, um einen hohen Wirkungsgrad dadurch zu erzielen, daß die Biomasse nacheinander im Druchgangsverfahren durch mehrere Behälter laufen muß.

Aus der DE-OS 31 02 739 ist ein Verfahren zur anaeroben Aufbereitung von pflanzlicher und tierischer Biomasse, auch von Abfall, bestehend aus organischen Materialien mit einem relativ hohen Molekulargewicht bekannt, bei dem in dem Reaktorraum eine Aufteilung von Säurephasen und Metanphasen in umgekehrten Verhältniss zu den Apparaten der Pozeßstufen Säure + Essigsäurebildung/Methanbildung vorzugsweise von etwa 1:10 erfolgt, wobei in dem abgeteilten Raum für die Säurephase die abgebauten Produkte nach unten sinken, und durch eine offene Verbindung in den Firmentationsraum für die Metanbildung gelangen.

Dieses bekannte Verfahren weist den Nachteil auf, daß die einzuführende Biomasse vor der Einführung in den Reaktionsbehälter auf etwa 30$^{o}$ Celsius erhitzt werden muß, da die offenbarte Vorrichtung selber

- 2 -

0158213

keine Heizeinrichtung umfaßt. Ferner weist diese Vorrichtung den Nachteil auf, daß die mehr oder weniger vergorene Biomasse mittels einer Pumpe über ein Austragrohr ausgepumpt werden muß, die wie man aus den Figuren dieser Druckschrift sieht, hoch über die Zuführungsleitung hinausragt. Hinzu kommt der Nachteil, daß wegen des Abpumpens an im wesentlichen nur einer, willkürlichen oder zufälligen Stelle in dem Gärbehälter, ein Teil der Biomasse bereits nach einer kurzen Verweilzeit abgepumpt wird, während andere Teile der Biomasse um ein vielfaches länger in dem Gärbehälter verbleiben, unabhängig davon, ob die Teile der Biomasse bereits vergoren sind oder nicht. Es ist klar, daß bei so unterschiedlichen Verweilzeiten, die, wie eine Betrachtung der Figuren dieser Offenlegungsschrift zeigt, durchaus um den Faktor 10 differrieren können, eine gleichmäßige Vergärung der eingebrachten Biomassen nicht möglich ist. Zwar ist bei der Vorrichtung dieser DE-OS 31 02 739 auch die Möglichkeit vorgesehen, den unteren Teil des Austragrohrs um einen Winkel von etwa 140 Grad zu schwenken. Diese Verschwenkungsmöglichkeit des Austragsrohrs ändert aber nichts an der Zufälligkeit der Auswahl der ausgetragenen Biomasse und an den um ein Vielfaches unterschiedlichen Verweilzeiten von verschiedenen Teilen der Biomasse.

Es sind noch eine Reihe anderer Verfahren und Vorrichtungen zur Erzeugung von Biogas bekannt, die jedoch durchweg den Nachteil aufweisen, daß die eingeführte Biomasse nicht gleichmäßig durch den Gärbehälter strömt und deshalb auch nicht gleichmäßig vergoren werden kann. Diese Vorrichtungen weisen im allgemeinem

- 3 -

Pumpeinrichtungen auf, welche die Biomasse im Gärbehälter vewirbelt, was einer gleichmäßigen Vergärung abträglich ist. Als Beispiele für derartige Vorrichtung seien die DE-OS 30 07 396, die DE-OS 32 11 888 und die DE-OS 29 14 802 genannt. Ferner wird auf den Aufsatz "Technische Neuentwicklungen landwirtschaftlicher Biogasanlagen" hingewiesen, der in der Zeitschrift "Korrespondenz Abwasser" im Heft 6, 1983 auf den Seiten 406 bis 414 abgedruckt ist und in dem verschiedenen Biogasanlagen beschrieben sind, welche ebenfalls die genannten Nachteile aufweisen.

Aufgabe der vorliegenden Erfindung ist es, ein Verfahren zur Erzeugung von Biogas zu schaffen, welches die Nachteile der bekannten Verfahren vermeidet und insbesondere eine gleichmäßige Vergärung von Biomasse mit hohem Wirkungsgrad zur Erzeugung von Biogas ermöglicht.

Mit der Erfindung wird zunächst der große Vorteil erzielt, daß die langsam einlaufende, frische Biomasse während sie von oben her durch eine Leitung in den Gärbehälter fließt, von einem Teil der im Behälter befindlichen Biomasse vorgewärmt wird.

Ein weiterer wesentlicher Vorteil der Erfindung ist daran zu sehen, daß die in den Ringraum des Behälters eingeleitete frische Biomasse nicht verrührt werden muß, sondern still und gleichmäßig vergären kann. Der Gasaustritt und die ständige Zu- und Abführung von Materie in Verbindung mit einer gezielten Wärmezuführung optimieren den als Thermik und Schwerkraft bezeichneten Ablauf.

Ein besonderer Vorteil der Erfindung liegt darin, daß die langsame Umwälzbewegung der zu vergärenden Masse durch die Erwärmung des eingeführten Materials auf einem Teil der Höhe des Ringraumes bewirkt wird, so daß die Biomasse besonders ungestört vergären kann und somit ein hoher Wirkungsgrad erzielt wird.

Um die Gleichmäßigkeit der Umwälzung der Biomasse in dem Gärbehälter zu erhöhen, wird die frische Biomasse gleichzeitig in zwei, drei oder noch mehr Winkelbereichen im unteren Ringraum des Gärbehälters zugeführt.

Die Erfindung weist ferner den Vorteil auf, daß das erfindungsgemäße Verfahren auch mit Vorrichtungen der Eingangsgenannten Art durchgeführt werden kann, bei denen im Außenwandbereich des Gärbehälters eine Heizung vorgesehen ist.

Diese weitergehende Aufgabe ist gemäß der Erfindung dadurch gelöst, daß die frische Biomasse von oben her durch die vergärende bzw. vergorene Biomasse hindurch, dieser Wärme entziehend und sich dadurch vorwärmend, in den unteren Bereich eines äußeren Ringraums des Gärbehälters eingeführt wird, dort auf einem Teil der Höhe zusätzlich von seinem inneren Umfangsbereich her aufgeheizt wird, wodurch der langsame Aufstieg der Biomasse unterstützt wird, und daß die im inneren des Gärbehälters abgesunkene Biomasse aus dem unteren Bereich des Gärbehälters durch Rohrleitungen abgeführt wird.

Von besonders erheblichem Vorteil ist bei den vorliegenden Erfindungen die Tatsache, daß die frisch eingeführte Biomasse im unteren Teil des Teilraumes des Gärbehälters nicht nur von der Außenwand, sondern auch von der zusätzlichen Heizung aufgeheizt wird. Durch dieses erfindungswesentliche Merkmal wird zum einen eine wesentlich gleichmäßigere und gleichförmigere Aufheizung der Biomasse ermöglicht, um diese auf den für die Vergärung optimalen Temperaturbereich einzustellen. Zum anderen weist dieses Merkmal der Erfindung auch den großen Vorteil auf, daß die langsame und gleichmäßige Aufsteigbewegung der vergärenden Biomasse gefördert wird. Im oberen Bereich des Gärbehälters driftet die aufgestiegene Biomasse, die weitgehend oder vollständig vergoren ist, zum inneren Bereich des Gärbehälters, und dort sinkt sie zu Boden; Aufgrund der Tatsache, daß die Biomasse im inneren Bereich des Behälters in wärmetauschender Verbindung mit der langsam zuströmenden, frischen Biomasse steht, die gemäß der Erfindung nicht vorgewärmt sein muß, wird die insbesondere im innersten Bereich des Gärbehälters absinkende Biomasse abgekühlt, wodurch weiterhin die thermische Umwälzbewegung der Biomasse unterstützt wird.

Auch aus diesem Grunde weist das erfindungsgemäße Verfahren einen ganz erstaunlichen hohen Wirkungsgrad bei der Erzeugung von Biogas auf.

Die zusätzliche Heizung bzw. die zusätzlichen Heizungen können eine geringere Temperatur als die Außenheizung aufweisen, da sie im inneren des Gärbehälters angeordnet
sind und die von ihnen erzeugte Wärme nicht nach
außen verloren gehen kann.

Es wird auch ausdrücklich darauf hingewiesen, daß
die Befüllung des Gärbehälters gemäß der Erfindung
sowohl kontinuierlich als auch diskontinuierlich
stattfinden kann. Es ist zum Beispiel möglich täglich
etwa eine halbe Stunde lang frische Biomassen nachzufüllen, wobei ein entsprechender Teil vergorener
Biomassen abgeführt wird.

Da die Aufsteigbewegung der vergärenden Biomasse
im wesentlichen durch gezielte thermische Einwirkung
verursacht wird, und wegen der besonderen Stromführung
ist die Geschwindigkeit der Aufsteigbewegung der
vergärenden Biomasse wesentlich geringer als die
der Absinkbewegung der vergorenen Biomasse. Es ist
zum Beispiel möglich, die Biomasse während eines
Zeitraums von etwa 30 Tagen langsam aufsteigen und
vergären zu lassen und sie gezielt innerhalb eines
Tages vom oberen Bereich des Gärbehälters bis zu
dem unteren Teil des Gärbehälters absinken zu lassen,
von welchem aus sie durch die Ableitungsrohre ausgetragen
wird.

Aufgabe der Erfindung ist es weiterhin, ein Verfahren
zur Erzeugung von Biogas zu schaffen, welches auch
bei Vorrichtungen ohne Außenwandbeheizung und unter
Verwendung geringerer Kosten für die Isolierung der
Außenwand des Gärbehälters möglich ist.

Diese weitergehende Aufgabe ist gemäß der Erfindung dadurch gelöst, daß die frische Biomasse durch die vergärende bzw. vergorene Biomasse hindurch, dieser Wärme entziehend und sie dadurch vorwärmend, in den unteren Bereich eines inneren Ringraumes des Gärbehälters eingeführt wird, dort auf einen Teil der Höhe des Ringraums sowohl von dessen innerem Umfangsbereich als auch zusätzlich vom äußeren Randbereich dieses inneren Ringraums her, der sich innerhalb des Gärbehälters befindet, aufgeheizt wird, wodurch der langsame Aufstieg der Biomasse unterstützt wird, und daß die Biomasse im äußeren Bereich innerhalb des Gärbehälters absinkt und aus dem unteren Bereich des Gärbehälters durch Rohrleitungen abgeführt wird.

Diese Ausführungsform der Erfindung weist besondere Vorteile hinsichtlich der thermischen Umwälzung der Biomasse auf. Da die frisch eingeleitete Biomasse im wesentlichen im zentralen Bereich des Gärbehälters aufsteigt und nach dem Gärvorgang im äußeren Randbereich des Gärbehälters wieder absinkt, ist eine gezielte Kühlung nicht nur möglich sondern automatisch erreicht. Das hat zum einen den Vorteil das Kosten zur Isolierung der Außenwand des Gärbehälters eingespart werden können, da diese Außenwand ja nicht besonders gut isoliert sein muß, sondern im Gegenteil die absinkende, vergorene Biomasse kühlen soll. Dadurch wird ja gerade die Absinkbewegung der vergorenen Biomasse beschleunigt, was erwünscht ist. Ferner wird in allen Varianten eine gezielte Ausnutzung der Schwerkraft erreicht.

Es wird an dieser Stelle noch darauf hingewiesen, daß die zusätzliche zweite oder mehrere zusätzliche Heizeinrichtungen um so wichtiger sind, je größer der Gärbehälter ist, weil die zu vergärende Biomasse im allgemeinen ein schlechter Wärmeleiter ist und bei Endlager-Vergärung eine Trennung von frischgärender und vergorener Materie erwünscht und notwendig ist.

Die vergorene Biomasse kann aus dem unteren Bereich des Behälters seitlich oder durch den Boden des Gärbehälters ausgetragen werden.

Nach einer anderen Weiterbildung der Erfindung wird die Biomasse aus dem unteren Bereich des Behälters durch eine Leitung im Gegenstrom zu der durch die Einführungsleitung relativ langsam einlaufenden, frischen Biomasse und in wärmeaustauschender Verbindung mit dieser nach oben geführt und von dort ausgetragen. Bei dieser Weiterbildung der Erfindung wird die zugeführte frische Biomasse besonders gut vorgewärmt.

Die frische Biomasse wird relativ langsam in den Gärbehälter eingeleitet, und sie befindet sich in der Regel zwischen 10 und 50 Tagen in dem Gärbehälter. Nach einer bevorzugten Ausführungsart der Erfindung befindet sich die Biomasse etwa 25 bis 32 Tage in dem Gärbehälter. Selbstverständlich wird im Rahmen der Erfindung die Verweildauer, die auch durch die Steuerung der Zuflußgeschwindigkeit der frischen Biomasse beeinflußt werden kann, auf die Besonderheiten der zu vergärenden Biomasse abgestimmt.

Aufgabe der Erfindung ist es ferner, eine Vorrichtung

0158213

zur Erzeugung von Biogas aus Biomasse mit einem besonders hohem Wirkungsgrad zu schaffen.

Diese Aufgabe ist bei Vorrichtungen zur Erzeugung von Biogas aus Biomasse ein Durchgangsverfahren, die eine in den unteren Bereich des Gärbehälters mündete Zuführleitung für frische Biomasse und eine Heizeinrichtung umfassen, dadurch gelöst, daß die Zuführungsleitung aus wärmeleitendem Material besteht, sich durch die vergärende bzw. vergorende Biomasse in den unteren Behälterraum erstreckt und sich von dort in mindestens zwei Rohrleitungen verzweigt, die einem Ringbereich des Gärbehälters münden, und daß Heizeinrichtungen zu beiden Seiten auf einem Teil dieses Ringbereichs, die frisch eingeführte Biomasse von zwei Seiten her aufwärmend, vorgesehen sind.

Diese Prinziplösung der Erfindung läßt es zunächst noch offen, wo im Gärbehälter diese im Ringbereich, in den die frische Biomasse eingeführt wird, sich befindet. Wie im folgenden noch beschrieben wird, gibt es zwei bevorzugte, deutlich voneinander unterschiedene Ausführungsformen.

Entscheidend ist jedoch, daß bereits bei dieser Prinziplösung die Strömungsbewegung durch die thermische Aufheizung der frisch eingeführten Biomasse verursacht wird. Die Biomasse muß somit nicht ständig verrührt werden, was eine Störung des Gärvorganges bedeutet. Da die Umwälzbewegung der Gärmasse insbesondere durch die thermische Aufheizung erfolgt, kann die Gärmasse

- 10 -

besonders gleichmäßig in ihre Umwälzbewegung versetzt werden, so daß ein besonders hoher Wirkungsgrad bei der Erzeugung von Biogas erzielt wird.

Ausgehend von der Vorrichtung der Eingangsgenannten DE-OS 30 10 183, bei welcher im Bereich der Außenwand des Gärbehälters eine Heizvorrichtung vorgesehen ist, ist die Aufgabe der Erfindung, eine Vorrichtung zur Erzeugung von Biogas zu schaffen, welche die Nachteile der bekannten Vorrichtungen vermeidet, und mit hohem Wirkungsgrad arbeitet, gemäß einer bevorzugten Weiterbildung der Erfindung dadurch gelöst, daß die Zuführungsleitung aus wärmeleitendem Material besteht, sich durch die vergärende bzw. vergorene Biomasse in den unteren Behälterraum erstreckt und sich von dort in mindestens zwei Rohrleitungen verzweigt, die in den äußeren Bereich des Gärbehälters münden, und daß etwa im mittleren Abstand zwischen Außenwand und Zentrum des Gärbehälters, im wesentlichen in dessen unteren Abschnitt eine oder mehrere zusätzliche Heizeinrichtungen vorgesehen sind.

Diese Ausführungsform der Erfindung weist insbesondere den Vorteil auf, daß sie eine besonders gleichmäßige Umwälzbewegung und damit eine besonders günstige Vergärung der Biomasse ermöglicht.

Ferner wird darauf hingewiesen, daß bei der erfindungsgemäßen Vorrichtung der Aufstieg der erwärmten Biomasse in einem wesentlichen größeren Volumen als die Absinkbewegung stattfindet, da die Fläche zum Beispiel eines Kreisringes mit dem Quadrat des Abstands vom Mittelpunkt wächst. Da die frische Biomasse in den äußeren Ringraum des Gärbehälters eingeleitet wird, findet der Aufstieg sehr langsam statt, so daß die Biomasse gleichmäßig vergären kann. Das Absinken dagegen findet im Bereich des Wärmeaustausches mit dem Zuführungsrohr statt, was eine gezielte Kühlung in einen kleineren Ringflächenraum zur Folge hat, so daß die vergorene Biomasse schneller absinken kann. Diese Zirkulation wird auch durch den Gasaustritt der vergärenden Biomasse selber gefördert.

Darüber hinaus weist die erfindungsgemäße Vorrichtung gegenüber sämtlichen bekannten Vorrichtungen den erheblichen Vorteil auf, daß die Wärmezufuhr zu der eingeführten frischen Biomasse besonders günstig ist, weil sie von zwei Seiten aus erfolgt, nämlich von der Außenwand des Behälters und von der inneren Zusatzheizung. Auf diese Weise ist eine schnelle und gleichmäßigere Erwärmung der schlecht leitenden Biomasse möglich.

Die Erfindung weist weiterhin den Vorteil auf, daß der Aufwand an kostenintensiven Bauteilen gering gehalten ist und die Herstellung preisgünstig möglich ist.

Vorzugsweise ist die Zuführungsleitung im wesentlichen vertikal im Zentrum des Gärbehälters angeordnet. Gerade diese zentrale Anordnung im Gärbehälter weist den Vorteil auf, daß die Umwälzbewegung der Biomasse, im äußeren Bereich des Gärbehälters nach oben aufsteigend zum zentralen Bereich des Gärbehälters driftend und dort absteigend, besonders gleichmäßig verläuft. Dieser Vorteil ergibt sich zwingend bereits aus der Symmetrie, die durch die zentrale Anordnung der Zuführungsleitung geschaffen wird.

Nach einer Weiterbildung der Erfindung ist die zusätzliche Heizung umlaufend angeordnet, vorzugsweise in einer umlaufenden Wand. Diese Weiterbildung der Erfindung trägt ebenfalls zur noch gleichmäßigeren Erwärmung der frischen Biomasse bei. Die zusätzliche Heizung kann eine Isolierung zur Hemmung oder Verringerung des Wärmeübergangs von ihr zu dem inneren Bereich des Gärbehälters aufweisen. Auf diese Weise ist eine noch intensivere Nutzung der zugeführten Wärme zur Aufheizung der frisch zugeführten Biomasse möglich.

Da die zusätzliche Heizung in dem Inneren des Gärbehälters angeordnet ist, kann sie auch dann, wenn sie eine geringere Temperatur und eine geringere Wärmeabgabeleistung als die Heizung in der Außenwand des Gärbehälters hat, eine effektive Vergärung der Biomasse und auch deren thermische Umwälzung einleiten und aufrechterhalten.

Die zusätzliche Heizung bzw. die zusätzlichen Heizungen sind nach einer anderen Weiterbildung der Erfindung von wärmespeicherndem Material umgeben. Diese Weiterbildung der Erfindung weist den Vorteil auf, daß auch dann, wenn die zusätzliche Heizung bzw. die zusätzlichen Heizungen zwischenzeitlich einmal ausfallen sollten, das wärmespeichernde Material noch Wärme

0158213

an die frische Biomasse abgibt. Selbst bei einer Unterbrechung der Arbeit der zusätzlichen Heizung bzw. der zusätzlichen Heizungen kann der Gärvorgang noch längere Zeit ungestört ablaufen, da selbst bei einem Ausfall dieser inneren Heizung während einer Zeit von zum Beispiel 3 Tagen die Temperatur im Bereich dieser Heizung lediglich um 1 bis 3 Grad abfällt. Dieser notfalls auftretende Temperaturabfall ist um so kleiner, je größer die Wärmespeicherkapazität des wärmespeichernden Materials ist.

Die Höhe der zusätzlichen Heizeinrichtung innerhalb des gesamten Gärbehälters ist variabel und kann insbesondere auf die Art der zu vergärenden Biomasse abgestimmt sein. Die Höhe dieser zusätzlichen Heizeinrichtung bzw. Heizeinrichtungen und der Abstand zur Außenwand richtet sich auch nach den Dimensionen des Gärbehälters insbesondere nach dessen Durchmesser.

Die zusätzliche Heizung erstreckt sich nach einer Ausführungsform der Erfindung von der Unterseite des Gärbehälters nicht höher als bis zu einer Teilhöhe des Behälters.

Nach einer bevorzugten Ausführungsform der Erfindung erreicht die zusätzliche Heizung von der Unterseite des Gärbehälters bis unter einem Drittel der Höhe des Gärbehälters.

Diese Ausführungsform ermöglicht eine besonders günstige Umwälzbewegung der Biomasse.

- 14 -

Nach einer Ausführungsform der Erfindung kann im inneren, unteren Bereich des Gärbehälters ein Austragsrohr vorgesehen sein, welches die abgesunkene Biomasse nach außen führt.

Nach einer anderen Weiterbildung der Erfindung ist im zentralen Bereich des Gärbehälters eine, mit der Zuführungsleitung in Wärmeaustausch stehende, vom unteren Ende des Gärbehälters nach oben führende Ableitung vorgesehen, von deren oberen Endbereich eine Austragleitung nach außen führt.

Diese Weiterbildung der Erfindung weist gleich zwei besondere Vorteile auf. Zum einem wird die frische Biomasse, die durch die Zuführungsleitung strömt, während sie langsam zugeführt wird, von der die Zuführungsleitung umgebenden Biomasse erwärmt. Diese Erwärmung findet sowohl bei einer kontinuierlichen Beschickung der Gäranlage also bei einer diskontinuierlichen Beschickung der Gäranlage statt. Diese Vorwärmung erspart zum einen Heizenergie und sie weist ferner den Vorteil auf, daß die frische Biomasse, wegen dieser zusätzlichen Aufwärmung, bereits für den Gärprozeß vorbereitet wird. Auf diese Weise werden längere Startzeiten in dem Ringbereich, in welchem die frisch eingeführte Biomasse auch von der Zusatzheizung aufgeheizt wird, auf ein Minimum reduziert.

Zum anderen weisen beide Weiterbildungen der Erfindung den Vorteil auf, daß die zur Vorwärmung der zugeführten frischen Biomasse erforderliche Heizenergie der direkt

- 15 -

die Zuführungsleitung umgebenden Biomasse entzogen wird. Da diese bereits vergoren ist, soll sie ohnehin absinken, damit sie über die Austragleitung ausgetragen werden kann. Auf diese Weise wird eine gezielte Kühlung der ohnehin nicht mehr erwünschten Gärmasse erreicht, wodurch die thermische Umwälzbewegung, unter Ausnutzung der Schwerkraft besonders günstig gestaltet wird.

Zweckmäßigerweise kann im zentralen Bereich des Gärbehälters eine die Zuführungsleitung umgebende aus dem unteren Bereich nach oben führende Mantelleitung vorgesehen sein, von deren oberen Endbereich eine Austragleitung nach außen führt. Diese Ausbildungsform der Erfindung begünstigt die Vorwärmung der zulaufenden Biomasse.

Diese Weiterbildung der Erfindung ermöglicht einen besonders günstigen Wärmeaustausch zwischen der vergorenen Biomasse, die durch den äußeren Teil der Mantelleitung ausgeführt wird, und der frisch eingeführten Biomasse. Auf diese Weise wird zwar auch die vergorene Biomasse gekühlt, die nicht mehr absinkt sondern aufsteigen soll. Das stört jedoch nicht, da die aufsteigende Biomasse der sie umgebenden, außerhalb der Mantelleitung im Gärbehälter befindlichen Biomasse wiederum Wärme entzieht, die dadurch immer noch gekühlt und in ihrer Absinkbewegung beschleunigt wird.

Die Austragleitung ist nach einer Ausführungsform der Erfindung außerhalb des Gärbehälters bis zu einer unteren Höhe des Gassammelringbereiches geführt, und dadurch wird die Höhe der Biomasse bis in den Gasbereich festgelegt.

Diese Weiterbildung der Erfindung bringt den Vorteil mit sich, daß die Biomasse den Gärbehälter vollständig ausfüllt und sich bis in den Gassammelbereich hinein erstreckt. Dadurch wird der Vorteil erzielt, daß die Verbindung zwischen Gassammelring und Oberseite des Gärbehälters nicht gasdicht sondern lediglich gegen Austritt der Biomasse in diesem Dichtungsbereich abgedichtet sein muß. Eine derartige Dichtung ist wesentlich einfacher und kostengünstiger herzustellen als eine Gasabdichtung.

Ein anderer Vorteil dieser Weiterbildung der Erfindung liegt daran, daß die gesamte Gäranlage besonderes rostfrei ausgebildet werden kann, was von großer Bedeutung ist, da Biogas besonders aggresiv ist. Diese günstige Ausführungsform ist möglich, weil lediglich der relativ kleine Gassammelring aus korosionsbeständigen Material hergestellt sein muß.

Die nach außen führende Austragleitung ist zweckmäßiger Weise in ihrer Höhe verstellbar. Auf diese Weise ist es möglich, den Pegel der Biomasse innerhalb des Gassammelringes zu verändern. Das ist insbesondere dann von Vorteil, wenn sich im Gassammelring besonders träges Material gesammelt haben sollte, das schlecht zirkuliert. In diesem Fall ist es möglich, bei sich ansammelnder Gasmenge und zunehmendem Gasdruck, die nach außen führende Austragleitung niedriger einzustellen, so daß der Pegel der Biomasse auf eine

- 17 -

Höhe unterhalb des Gassammelringes gesenkt werden kann. Dabei wird zuvor gefangenes Material aus dem Gassammelring entfernt, in den normalen Umwälzstrom eingeleitet und mit diesem mitgeführt.

Die Decke des Gärbehälters steigt zweckmäßiger Weise zur Gassammelstelle hin geringfügig an. Diese Weiterbildung der Erfindung hat den Vorteil, daß in dem Deckenbereich angesammeltes Biogas bei der Strömung des Biomaterials zum zentralen Bereich hin mit Sicherheit mitgeschleppt und damit der Transport des Biogases in den Gassammelring unterstützt wird. Zusätzlich wird eine Schwimmdeckenbildung ständig mit Gas selbsttätig durchperlt.

Nach einer anderen Weiterbildung der Erfindung ist im Gassammelringes oder im oberen Bereich des Gärbehälters eine verschließbare Abführleitung vorgesehen. Diese Weiterbildung der Erfindung weist den Vorteil auf, daß besonders leichtes Material, welches sich oben in Schwimmschichten angesammelt hat, wann immer es erwünscht ist, abgeleitet werden kann. Auf diese Weise ist selbst dann, wenn die eingeleitete Biomasse heterogen ist, eine gleichmäßige Umwälzung der Biomasse in dem Gärbehälter und damit ein gleichmäßiger Umlauf der Biomasse möglich ist, wodurch der Wirkungsgrad bei der Erzeugung von Biogas erhöht wird.

Der Gärbehälter ist nach einer bevorzugten Ausführungsform der Erfindung rund. Diese weiterbenannte Erfindung führt zu einer besonders gleichmäßigen Umwälzbewegung und damit zu einem besonders hohen Wirkungsgrad,

0158213

weil die Störungen beim Vergärungsprozeß minimal
sind.

Nach einer anderen Weiterbildung der Erfindung ist
eine Rührvorrichtung vorgesehen. Diese dient insbesondere
zum Austragen von Schwimmschichten und von Sinkschichten
und zu gelegentlichen Reinigungsarbeiten kann aber
auch während des Gärprozesses eingeschaltet sein
bzw. werden. Aus diesem Grunde ist die Rühr- oder
Pumpvorrichtung gemäß der Erfindung zweckmäßiger
Weise so ausgebildet, daß das im Gärbehälter enthaltene
Material im wesentlichen um den zentralen Bereich
herumgewälzt wird, ohne oder mit nur vernachlässigbar
kleinem vertikalen Materialaustausch. Der gelegentliche
oder auch häufigere Einsatz des Rührsystems ist besonderer bei der runden oder nahezu runden Ausführungsform des Gärbehälters von besonderer Wirksamkeit.

Auch bei Biogasanlagen, welche keine Heizung im Bereich
ihrer Außenwand aufweisen, läßt sich die Erfindung
verwirklichen. Diese Aufgabe ist bei Vorrichtung
zur Erzeugung von Biogas im Durchgangsverfahren,
mit einer in den unteren Bereich eines Gärbehälters
mündenden Zuführungsleitung dadurch gelöst, daß die
Zuführungsleitung aus wärmeleitendem Material besteht,
sich durch die vergärende bzw. vergorene Biomasse
in den unteren Behälterraum erstreckt und sich von
dort in mindest zwei Rohrleitungen verzweigt, die
in einen inneren Ringbereich des Gärbehälters münden,
und daß Heizeinrichtungen zu beiden auf einem Teil
der Höhe dieses Ringbereichs vorgesehen sind, welche
die Biomasse von zwei Seiten her aufwärmen bzw. auf-

- 19 -

heizen.

Diese Ausführungsform der Erfindung ermöglicht, wie auch die anderen Ausführungsformen, eine besonders gleichmäßige Umwälzbewegung der zu vergärenden Biomasse. Darüber hinaus weist sie den Vorteil auf, daß die Biomasse im Außenbereich absinkt und dabei von außen her gekühlt wird. Die erfindungsgemäße Vorrichtung erspart somit Kosten zur Isolierung der Außenwand des Gärbehälters, da die an der Innenseite der Außenwand des Gärbehälters befindliche Biomasse, die ja bereits weitgehend oder vollständig vergoren ist, absinken soll. Durch die Kühlung durch die Außenwand wird gezielt die bereits vergorene Biomasse abgekühlt, so daß die Thermik, unter Ausnutzung der Schwerkraft, gezielt gefördert wird.

Ferner weist diese Weiterbildung der Erfindung auch den Vorteil auf, daß die von den beiden Heizeinrichtungen, welche die frisch eingeführte Biomasse von zwei Seiten aufheizen, zugeführte Wärme vollständig in den Gärbehälter eingeführt wird.

Bei dieser Ausführungsform kann, wie bei den oben beschriebenen Weiterbildung der anderen Ausführungsformen, die Zuführungsleitung im wesentlichen im Zentrum des Gärbehälters angeordnet werden. Im übrigen kann diese andere Ausführungsform der Erfindung in ähnlicher Weise wie die oben beschriebene Ausführungsform weitergebildet werden, und deshalb kann hinsichtlich Einzelheiten der möglichen Weiterbildungen im wesentlichen verwiesen auf die obigen Ausführungen werden kann.

Wie bereits erwähnt, kann die Befüllung der erfindungsgemäßen Vorrichtungen sowohl kontinuierlich als auch
diskontinuierlich vorgenommen werden.

Darüber hinaus ist aber auch eine andere Verwendungsform
der erfindungsgemäßen Vorrichtungen, und zwar der
verschiedenen Ausführungsformen möglich, nämlich
die Verwendung als Endlager-Gärvorrichtung. Auch
zu diesem Verwendungszweck kann die Biomasse
kontinuierlich, sie kann aber auch diskontinuierlich
zugeführt werden. Der entscheidende Unterschied ist
jedoch, daß die Entnahme nach erheblichen Lager-
oder Gärzeiten stattfindet. Der wesentliche Unterschied
dieser Ausführungsformen zu den oben beschriebenen
besteht darin, daß jetzt lediglich eine gasdichte
Abdeckung vorgesehen sein muß, welche einen Lufteintritt
zu dem nur teilweise gefüllten Gärbehälter verhindert.
Wenn der Behälter weitgehend leer ist, wird diese
Gasabdeckung sich in einer sehr tiefen Lage befinden.
Diese Gasabdeckung wird aber im allgemeinen nicht
auf der vergärenden Masse aufliegen, sondern sich
darüber befinden, weil sie gleichzeitig einen Gassammelbehälter nach oben hin begrenzt. Diese Gasabdichtung bildet zusammen mit Teilen der Außenwand
und mit der zu vergärenden Masse bzw. mit der Unterseite
des Gärbehälters einen Gassammelbehälter. Die Gasabdeckung selber kann vorzugsweise eine Folie aus
PVC oder aus einem gummierten Gewebe sein.

Vorzugsweise ist diese Folie am Außenwandbereich des Gassammelbehälters befestigt. Ferner ist die Folie im oberen Bereich der Einführrohre mit diesen, bzw. mit der umgebenden Mantelleitung, gasdicht abgeschlossen. Dieser Bereich unterhalb der Verbindungsstelle ist als Gassammelbereich ausgebildet, weil er der höchste Bereich ist. Die Befestigung der Folie am Außenwandbereich ist vorzugsweise tiefer als diejenige am Einfüllbereich.

Die Folie fällt deshalb im allgemeinen vom zentralen Einfüllbereich zum Außenwandbereich der Anlage hin ab. Dieser Abfall ist um so stärker, je weniger Gas und je weniger Biomasse in der Gärendlage enthalten sind.

Bei dieser Ausführungsform der Erfindung ist aus folgenden Gründen stets eine Startgärung oder Startmasse in dem Behälter enthalten: Wie oben beschrieben wurde wird die frische Biomasse in einen äußeren bzw. in einen inneren Ringbereich eingeführt, in welchem sie von zwei Seiten erwärmt wird. Erst wenn der Füllstand der Biogasanlage über die Höhe dieser zusätzlichen Heizeinrichtung, die als umlaufende Wand ausgebildet ist, ansteigt, wird die teilweise vergorene Biomasse vom äußeren Ringbereich zum zentralen Bereich hin bzw. umgekehrt abfließen können. Aus diesem Grunde ist selbst dann, wenn vergorene Biomasse aus dem nicht zusätzlich beheizten Ringbereich abgeführt wird, der beheizte Ringbereich stets gefüllt.

Die Durchbiegung der Folie nach unten bzw. ihre Auswölbung nach oben hängt im wesentlichen von der noch

0158213

vorhandenen Gasmenge ab, welche so lange in diesem Endlager gespeichert werden kann, bis sie verbraucht wird. Auf diese Weise stellt die Gäranlage gleichzeitig einen Gasspeicher dar.

Das Volumen zwischen der Gasfolie und der Deckenseite des Gärbehälters wird hinterlüftet, so daß die Bewegung der Gasfolie selber mit zunehmendem Gasvolumen nicht behindert wird.

Der erwärmte Bereich ist bei dieser Ausführungsform stets mit Biomasse gefüllt. Der nicht zusätzlich beheizte Bereich stellt bei dieser Ausführungsform ein Endlager dar, dessen Volumen entsprechend der Höhenlage der Folie flexibel ist.

Mit dieser Ausführungsform der Erfindung ist es möglich, Biomasse über den ganzen Winter hin kontinuierlich oder diskontinuierlich langsam einzufüllen und sie, falls erwünscht, über Monate in dem kühleren Bereich nachgären zu lassen.

Diese Ausführungsform der Erfindung hat eine der Jahresverbrauchkurve des Gases anpaßbare Gasproduktion. Im Winter ist dieses Endlager im wesentlichen mit Biomasse gefüllt, so daß aufgrund der größeren Masse eine große Gasproduktion auch bei geringerem Temperaturniveau stattfindet. Im Sommer, wenn weniger Biomasse zum Vergären zur Verfügung steht, wird aufgrund der höheren Umwelttemperatur ein höheres Temperaturniveau gefahren werden, wodurch eine kürzere Vergärzeit große Mengen an Biogas liefert.

- 23 -

0158213

Weitere Vorteile und Merkmale der Erfindung gehen aus den Unteransprüchen in Verbindung mit der Beschreibung und der Zeichnung hervor. In letzter zeigen:

Fig. 1 einen Querschnitt durch eine Ausführungsform der Erfindung, bei welcher die vergorene Biomasse im oberen Bereich des Gärbehälters ausgetragen wird,

Fig. 2 eine andere Ausführungsform der Erfindung, bei welcher die vergorene Biomasse aus dem unteren Teil des Gärbehälters abgezogen wird,

Fig. 3 eine weitere Ausführungsform der Erfindung,

Fig. 4 eine Ausführungsform der Erfindung, bei welcher die Biomasse in im wesentlichen umgekehrter Richtung in dem Gärbehälter umgewälzt wird,

Fig. 5 eine Ausführungsform der Erfindung, bei welcher der Biogasbehälter als Endlager ausgebildet ist, und

Fig. 6 eine als Endlager verwendbare Ausführungsform der Erfindung, bei welcher die zusätzlichen Heizungen gleichzeitig zur Stützung der Decke des Gärbehälters dienen.

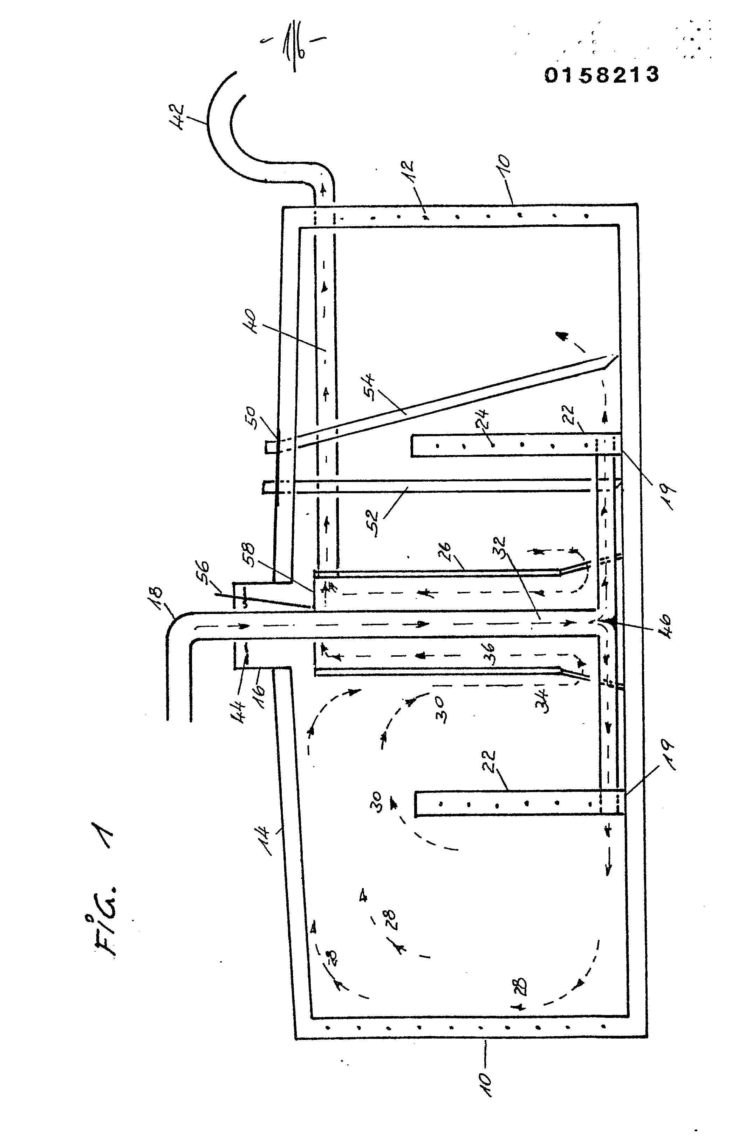

In Figur 1 sieht man einen Biogasbehälter, in dessen Außenwänden 10 Heizungsrohre 12 vorgesehen sind.

- 24 -

Der Biogasbehälter weist eine Decke 14 auf, die zum zentralen Bereich hin leicht ansteigt und dort eine Ausnehmung aufweist, die den Weg zu einem Gassammelring 16 freigibt, aus welchem das erzeugte und gesammelte Biogas in bekannter Weise abgeführt werden kann.

Durch den zentralen Bereich des Gassammelringes 16 erstreckt sich eine Zuführungsleitung 18, welche von oben beschickt wird und zugeführte Biomassen zunächst, wie es durch die Pfeile angedeutet ist, in den unteren zentralen Bereich des Gärbehälters einleitet. Dort verzweigt sich die Zuführungsleitung 18 in zwei Leitungen 19 und 19', welche sich horizontal vom zentralen Bereich des Gärbehälters fort zu dessen äußerem Bereich hin erstrecken und in einen äußeren Ringbereich münden. Dieser äußere Ringbereich wird zum einen durch die Außenwand 10 und zum anderen durch eine innere Wand 22 begrenzt, welche als umlaufende Ringwand ausgebildet ist. Auch die innere Wand 22 weist Heizrohre 24 auf. Durch die Heizeinrichtungen 12 der Außenwand 10 und 24 der Innenwand 22 wird die frisch zugeführte Biomasse im äußeren Ringraumbereich 26 von zwei Seiten her gleichzeitig aufgeheizt. Diese gleichzeitige Aufheizung von zwei Seiten ist besonders wichtig, weil das Biomaterial im allgemeinen ein schlechter Wärmeleiter ist.

Wie durch die Pfeile 28, 28' und 28'' angedeutet ist, steigt die erwärmte Biomasse, während sie gleichzeitig gärt, in dem Behälter auf. Im oberen Bereich driftet sie, durch den Gasaustritt unterstützt, in den zentralen Bereich, und dort sinkt sie wie durch den Pfeil 30 angedeutet ist, nach unten in den zentralen Bereich ab.

In diesem zentralen Bereich ist eine Mantelleitung 32 vorgesehen, welche die Zuführungsleitung 18 umfaßt. Diese Mantelleitung 32 ist unten geöffnet, wie es bei 34 dargestellt ist, so daß die abgesunkene Biomasse in die Mantelleitung 32 hinein fließen kann. In dieser Leitung 32 wird sie, wie durch den Pfeil 36 angedeutet ist, nach oben transportiert, und im oberen Bereich dieser Mantelleitung ist ein Austragrohr 40 angesetzt, über welches die vergorene Biomasse ausgetragen wird.

Dieses Austragrohr 40 ist außerhalb des Gärbehälters über die Oberseite des Gärbehälters hinaus geführt, vergleiche den Teil 42 der Austragleitung 40. Durch die Führung dieses Bereichs 42 der Austragleitung wird die Höhe der Biomasse in dem Gassammelring 16 bestimmt, wie es durch die nur symbolisch angedeutete Oberfläche bei 44 erkennbar ist.

Zur Verbesserung der Wirkungsweise der beschriebenen Ausführungsform ist ein Verteilkeil oder Kegel 46 im unteren Ende der Zuführungsleitung 18 vorgesehen, welcher verhindert, daß sich in dem Bereich Ablagerungen bilden.

Ferner ist bei dieser Ausführungsform eine Steuerstange 56 vorgesehen, welche sich von oben durch den Gassammelring 16 bis zu einer Klappe 58 erstreckt. Diese Klappe 58 ist normalerweise geschlossen und wird
üblicherweise beim Gärprozeß nicht benötigt. Falls
sich jedoch im oberen Bereich des Gärbehälters Schwimmschichten ausbilden sollten, welche nicht mehr mit
der Hauptgärmasse nach unten absinken, ist es mit
Hilfe der Steuerstange 56 möglich, die Klappe 58
zu öffnen. In diesem Falle können die Schwimmschichten
durch die Austragleitung 40 ausgetragen werden.

Durch die bogenförmige Krümmung 42 des Austragrohrs
40 soll angedeutet sein, daß das Austragrohr höhenverstellbar ist.

Bei der Ausführungsform der Figur 1 sieht man deutlich,
daß der Pegel 44 der Biomasse sich in dem Gassammelring
16 befindet. Deshalb muß der Übergang zwischen der
Decke 14 des Gärbehälters und dem Gassammelring 16
nicht gasdicht sondern nur gegen den Austritt des
Biomaterials abgedichtet sein.

Wie man aus der Figur 1 sieht, wird die eingeführte
frische Biomasse, welche sich in dem Rohr 18 befindet,
von der auszutragenden Biomasse, die sich in dem
Mantelrohr 32 befindet, vorgewärmt. Dadurch wird
die in dem Mantel 32 enthaltene Biomasse ihrerseits
abgekühlt und wirkt damit kühlend auf die Biomasse,
welche das Mantelrohr 32 umgibt und sich abwärts
bewegt, wie es durch den Pfeil 30 angedeutet ist.

Selbstverständlich ist es auch möglich die Vorrichtung
so zu verwenden, daß die frische Biomasse nicht durch
die zentrale Zuführungsleitung 18 sondern vielmehr

0158213

durch eine Mantelleitung 32 eingeführt wird, wenn diese sich im unteren Bereich des Gärbehälters nach außen hin verzweigt und in den äußeren Ringbereich mündet. Bei einer derartigen Ausgestaltung der Biogasvorrichtung würde dann zwangsläufig die vergorenen Biomassen durch die zentrale Leitung 18 nach außen abgeführt werden. Diese Ausbildungsform der Erfindung hätte den weiteren Vorteil, daß die frisch eingeleitete Biomasse noch stärker vorgewärmt und die innerhalb des Gärbehälters in Richtung des Pfeiles 30 absinkende Biomasse noch stärker, und zudem gezielt, gekühlt würde. Dadurch würde die Abwärtsbewegung der Biomasse innerhalb des Behälters noch stärker gefördert werden, und damit der gesamte thermische Bewegungsablauf.

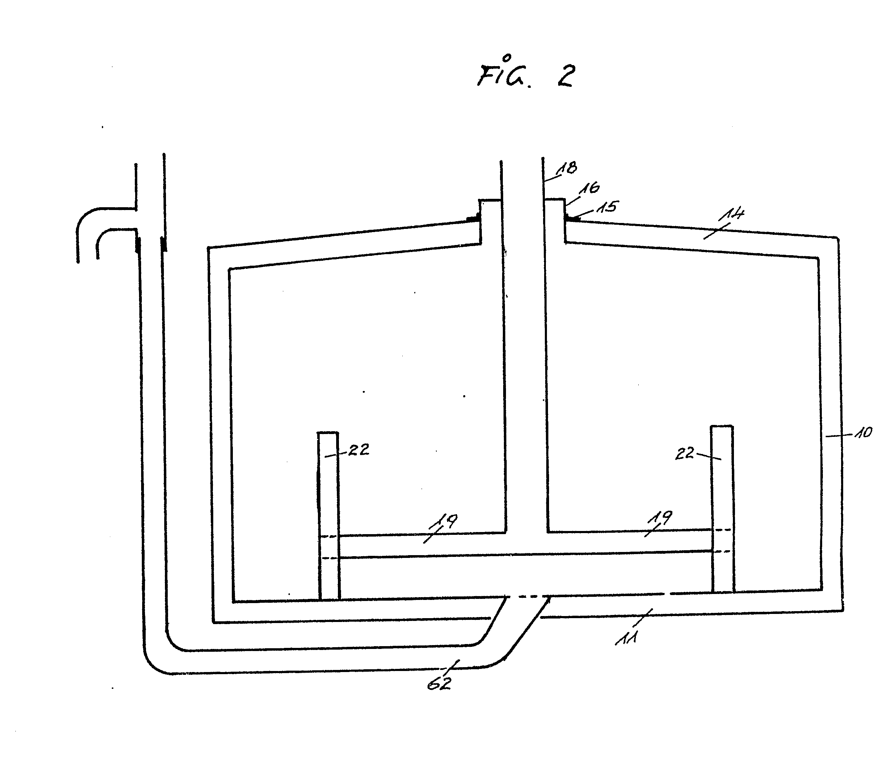

Eine leicht modifizierte Ausführungsform der Erfindung ist in Figur 2 dargestellt. Bei dieser Ausführungsform tragen gleiche Teile die selben Bezugszeichen wie in Figur 1. Der besseren Übersicht halber und um lediglich den Unterschied gegenüber der Ausführungsform der Figur 1 besser hervor treten zu lassen, sind eine Reihe von Details in dieser Figur 2 nicht aufgeführt. Der wesentliche Unterschied der Ausführungsform der Figur 2 gegenüber derjenigen der Figur 1 besteht darin, daß im unteren Bereich des Gärbehälters eine Austragleitung 62 vorgesehen ist, welche die vergorene Biomasse nach unten durch den Boden 11 des Gärbehälters abführt und sie seitlich neben dem Gärbehälter bis zur gewünschten Höhe führt. Diese Höhe ist wiederum entsprechend der gewünschten Einstellhöhe der Biomasse in dem Gärbehälter bzw. in dem Gassammelring 16 ein-

- 28 -

stellbar.

Ferner sieht man in dieser Figur 2 schematisch angedeutet eine Dichtung 15, welche die Verbindung zwischen
dem Gassammelring 16 und der Oberseite der Decke
14 des Gärbehälters abdichtet.

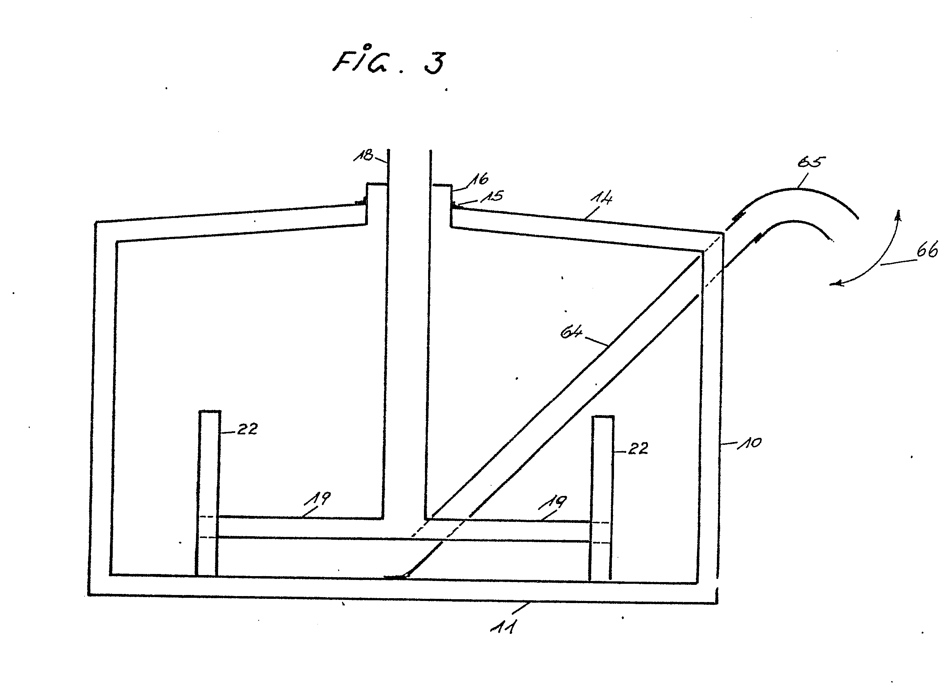

Eine andere Ausführungsform dieser Erfindung ist
in Figur 3 dargestellt. Diese Ausführungsform unterscheidet sich von derjenigen der Figur 2 im wesentlichen
dadurch, daß ein Austragrohr 64 vorgesehen ist, welches
aus dem unteren zentralen Bereich des Gärbehälters
diagonal zum rechten oberen Bereich des Gärbehälters
geführt und dort, selbstverständlich gegen die umgebenden Wände abgedichtet, durch die Wand 10 durchgeführt
ist. Durch den Doppelpfeil 66 dieser Figur 3 ist
angedeutet, daß der Endbereich 65 dieser Austragleitung
64 in seiner Höhe verstellbar sein soll. Auch diese
Ausführungsform der Erfindung weist den Vorteil auf,
daß die durch die Zuführungsleitung 18 eingeführte
Biomasse, wie auch beim Ausführungsbeispiel der Figur
2, über die wärmeleitende Wand der Zuführungsleitung
18 direkt mit der sie umgebenden Biomasse in wärmeaustauschender Relation steht. Dadurch wird die vergorene
Biomasse im innersten Bereich des Gärbehälters, nämlich
direkt von der Zuführungsleitung 18 aus, gezielt
gekühlt, wodurch die thermische Umwälzbewegung der
Biomasse unter Ausnutzung der Schwerkraft gefördert
wird.

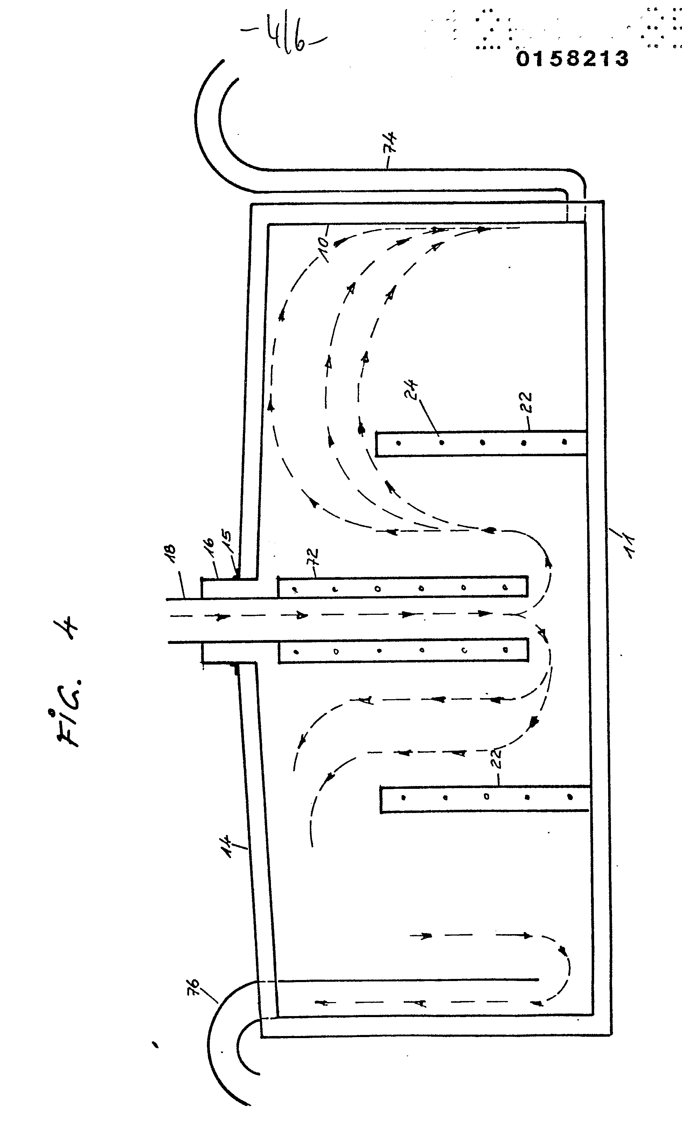

Die Figur 4 zeigt eine Ausführungsform der Erfindung,
bei welcher die eingeführte Biomasse in anderer Weise,

0158213

nämlich etwa in umgekehrter Richtung durch den Gärbehälter strömt. Um die zentrale Zuführungsleitung
18 ist bei dieser Ausführungsform ein aus wärmespeicherndem Material bestehender Heizmantel oder eine Heizmatte
70 vorgesehen, innerhalb derer sich Heizrohre 72
befinden. Bei dieser Ausführungsform wird die frisch
zugeführte Biomasse bereis in der Zuführungsleitung
18 von dieser Heizungswand 70, 72 vorgewärmt. Die
frisch eingeführte Biomasse wird durch die Zuführungsleitung 18 nach unten geführt und strömt dort in
einen inneren Ringbereich, welcher durch eine umlaufende
Wand 22 begrenzt ist, in der, wie in den anderen
Ausführungsbeispielen, Heizungsrohre 24 vorgesehen
sind. Auch in diesem Bereich wird die eingeführte
Biomasse von zwei Seiten her aufgeheizt. Dadurch
wird der Gärvorgang gefördert und die Biomasse steigt
im inneren Bereich des Gärbehälters nach oben. Von
dort driftet sie in dem äußeren Bereich des Gärbehälters
und sinkt, wie es in der rechten Hälfte der Figur
4 gezeigt ist, an der Außenwand bzw. im Bereich der
Außenwand 10 des Gärbehälters innerhalb desselben
nach unten. Im unteren Randbereich ist eine Austrageinrichtung 74 vorgesehen, welche die vergorene Biomasse
seitlich aus dem Gärbehälter heraus und neben dem
Gärbehälter nach oben hin abführt.

Diese Ausführungsform hat den Vorteil, daß die vergorene Biomasse an der Außenseite des Gärbehälters
gekühlt wird. Auf diese Weise werden Kosten für die
Isolierung der Außenwand gespart. Gleichzeitig wird
eine gezielte Kühlung der vergorenen Biomasse und
damit eine bessere thermisch verursachte Umwälzung

- 30 -

erzeugt.

Es ist auch möglich, wie es in der linken Seite der
Figur 4 symbolisch angedeutet ist, eine Austragleitung
67 innerhalb des Gärbehälters in dessen Außenwandbereich
vorzusehen.

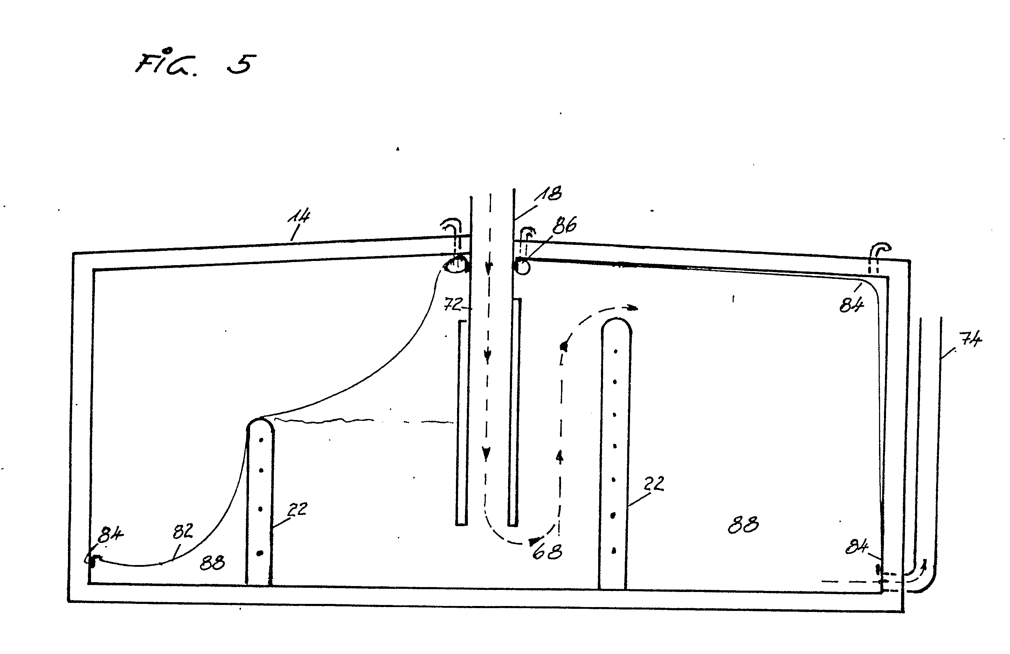

Die Figur 5 zeigt eine Ausführungsform der Erfindung,
bei welcher der Gärbehälter gleichzeitig als Endlager
verwendet wird.

Der besseren Übersichtlichkeit halber, und um lediglich
den wesentlichen Unterschied zu den anderen Ausführungsformen der Erfindung hervorzuheben, sind in dieser
Figur 5 Einzelheiten fortgelassen oder nur schematisch
angedeutet.

Der wesentliche Unterschied dieser Ausführungsform
gemäß der Figur 5 besteht darin, daß in dem Gärbehälter
eine Folie 82 vorgesehen ist, welche den unteren
Teil des Gärbehälters luftdicht von dem oberen Teil
des Gärbehälters abschließt. Diese Folie ist im Außenwandbereich des Gärbehälters bei 84 befestigt, und
ihr innerer Ringbereich ist bei 86 über einen Kragen
luftdicht mit der Zuführleitung 18 verbunden.

Wie man aus dieser Figur 5 sieht, handelt es sich
bei einer Ausführungsform, bei welcher der innere
Ringbereich 68 von der Heizung 72 und der zusätzlichen
Heizung 22 beheizt wird. Die Folie 82 bzw. 84 ist
in dieser Figur 5 in zwei Zuständen dargestellt.

In der linken Hälfte der Figur 5 ist der Fall dargestellt, daß der Gärbehälter relativ leer ist. In diesem Falle ist der innere, erwärmte Gärbereich bis zur Höhe der zusätzlichen Wand 22 gefüllt, wie es durch die Pegellinie 86 angedeutet ist. Der äußere Bereich 88 ist weitgehend leer. In diesem Fall wird die Folie 82, wie es in der linken Seite der Figur angedeutet ist, von ihrer zentralen Befestigung 86 hin im Bereich der Außenkante der Wand 22 an dieser anliegen und in einem Bogen zu der Außenwandbefestigung 84 hin durchhängen.

In der rechten Hälfte der Figur ist ein Zustand dargestellt, in welchem die Folie, die in diesem Falle, zur besseren Unterscheidung, mit 84 bezeichnet ist, sich weitgehend in ihrer nach oben durchgewölbten Lage befindet. Diese Lage ist zum einen durch die Höhe des Füllstandes in dem Endlagerbereich 88 und zusätzlich durch die Menge des Biogases bestimmt.

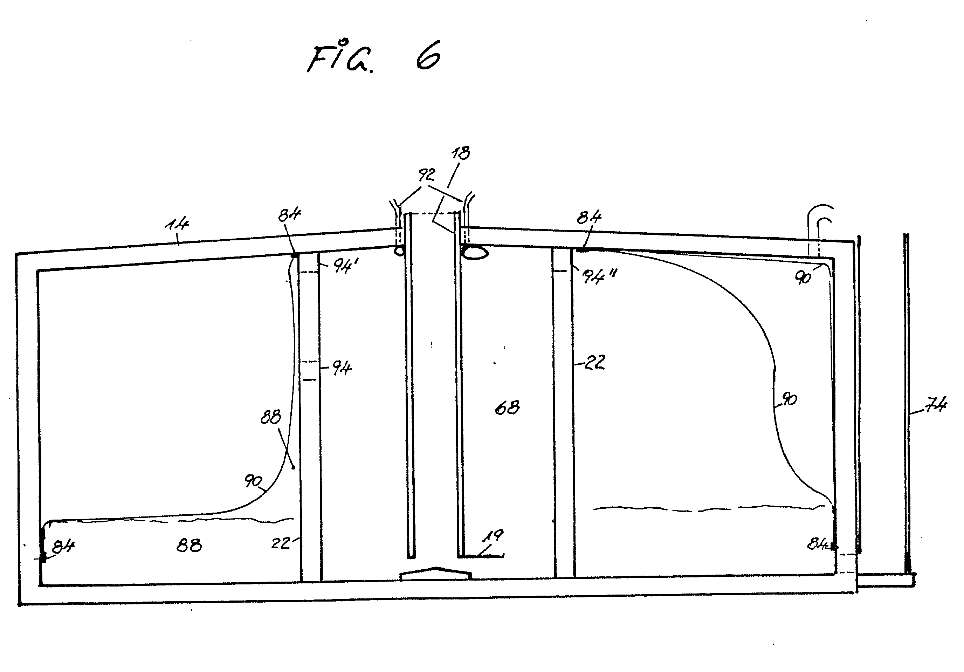

Die Figur 6 zeigt eine insbesondere als Endlager verwendbare Ausführungsform der Erfindung. Auch in dieser Figur 6 sind die Teile, die bereits in den anderen Ausführungsbeispielen vorhanden sind, mit gleichen Bezugszeichen versehen. Ferner sind, der besseren Übersichtlichkeit wegen, Einzelheiten weggelassen oder nur schematisch angedeutet, um lediglich den Unterschied zu den anderen Ausführungsbeispielen der Erfindung hervorzuheben.

In dieser Figur 6 sieht man am unteren Ende der Zuführungsleitung 18 auf der linken Seite ein Verteilrohr 19, während auf der rechten Seite kein Verteilrohr
angedeutet ist. Ferner ist unterhalb des Rohres 18
eine Verteilplatte 94 vorgesehen. Durch diese schematischen Zeichnungen soll ausgedrückt sein, daß sowohl
Verteilrohre 19 vorgesehen sein können, als auch
lediglich eine unterhalb der Zuführungsleitung 18
angeordnete Verteilplatte 94. Die Verteilplatte 94
kann nach außen hin auch abfallen, so daß sie in
ihrer Form einem Verteilkeil etwas ähnlicher wird.

Ein auffälliger Unterschied dieser Ausführungsform
der Figur 6 gegenüber derjenigen der Figur 5 liegt
darin, daß die zusätzlichen Heizungen 22 so ausgebildet
sind, daß sie gleichzeitig die Decke 14 des Gärbehälters
stützen. Zu diesem Zweck sind bei dem Ausführungsbeispiel
der Figur 6 die die zusätzlichen Heizungen enthaltenden
Wände 22 vom Bodenbereich des Gärbehälters bis zu
der Decke 14 hin durchgezogen und bilden gleichzeitig
Stützwände oder einen tragenden Zylinder. Damit die
vergorene Biomasse aus dem aufgeheizten inneren Teilbereich 68 austreten kann, sind in dieser Zylinderwand
22 Austrittsöffnungen 94, 94', 94'' vorgesehen.

In der linken Hälfte der Figur 6 ist die Ausführungsform
angedeutet, bei der mehrere Austrittsöffnungen 94,
94' vorgesehen sind, die jeweils in verschiedenen
Höhen angeordnet sind. Wenn die eingeführte Biomasse
die Höhe der ersten Austrittsöffnung 94 erreicht,
wird sie aus dem aufgeheizten inneren Bereich 98
durch die unterste Austrittsöffnung 94 nach links
und damit nach außen in den Bereich 88 hinüberfließen,
der bei dieser Ausführungsform als Nachgärraum dient.

- 33 -

Gleichzeitig dient dieser Raum 88 auch als Endlagerraum, dessen Volumen durch die bewegbar angeordnete Gasfolie 88 veränderlich ist. Wenn zunehmend mehr Biomasse eingeführt wird, so daß auch der Nachgärraum 88 bis zur oberen Höhe der Austrittsöffnung 94 angefüllt ist, wird, bei weiterer Zuführung von Biomasse, sowohl in dem Hauptgärraum 68 als auch in dem Nachgärraum 88 der Füllpegel weiter ansteigen. Die oberste Austrittsöffnung 94' dieser Ausführungsform befindet sich ganz oben an der Heizwand 22, so daß direkt bis an die Unterseite der Decke 14 angrenzend eine Austrittsöffnung vorgesehen ist. Diese Austrittsöffnung 94' ermöglicht ein Durchströmen des Gases aus dem Nachgärraum 88 zu dem zentralen Bereich des Gärbehälters, in dem, lediglich schematisch angedeutet, eine Gasabführeinrichtung 92 vorgesehen ist, die z. B. als Gassammelring oder Gassammelsack ausgebildet sein kann.

Auf der rechten Seite der Figur 6 ist eine andere Ausführungsform der Erfindung dargestellt, bei welcher die zylindrisch ausgebildete Heizwand 22 lediglich eine Gasaustrittsöffnung bzw. mehrere Gasaustrittsöffnungen 94'' aufweist, die alle im Bereich der Decke 14 in der zylindrischen Wand 22 ausgebildet sind.

Diese Ausführungsform der Figur 6 weist ferner den Unterschied zu derjenigen der Figur 5 auf, daß die Gasauffangfolie 88 bzw. 90 direkt an der Decke 14 des Gärbehälters befestigt ist. Das wiederum bringt den Vorteil mit sich, daß die Gasauffangfläche längs einer größeren Breite befestigt sein kann und somit stärker gegen Gewichts- und Zugbelastungen gesichert ist. Ferner ist bei dieser Ausführungsform der Figur 6 die Gasauffangfolie 88 bzw. 90 außerhalb der zylindrischen Heizungswand 22 gasdicht an der Decke 14 befestigt, also nicht notwendigerweise im zentralen Bereich des Gärbehälters.

Insbesondere bei der Ausführungsform der Figur 6 ist es auch möglich, die frisch eingeführte Biomasse lediglich von der zusätzlichen Heizung 22 aus zu beheizen und trotzdem die thermisch gesteuerte, gleichmäßige Umwälzbewegung der Biomasse während des Gärvorganges zu bewirken. Zu diesem Zweck ist die zusätzliche Heizung 22 zweckmäßigerweise so ausgebildet, daß sie nach außen isoliert ist, so daß sie ganz überwiegend nur nach innen hin Wärme abgibt und somit nur den inneren Hauptgärbereich 68 erwärmt. Diese Ausführungsform gilt vorzugsweise dann, wenn der Durchmesser des Hauptgärbereichs 68 relativ klein ist. Es wird jedoch darauf hingewiesen, daß ein kleiner Durchmesser dieses Hauptgärbereichs, im Vergleich zu den anderen Ausführungsformen, besonders deshalb möglich ist, weil die Heizwand 22 und damit die zusätzliche Heizung vom Bodenbereich bis zu einer großen Höhe, in gewünschten Fällen sogar bis zur Decke 14 hin hochgezogen sein kann. Auf diese Weise wird von der zusätzlichen Heizung 22 trotz relativ kleinen Durchmessers des Hauptgärbereichs 68, wegen der größeren Höhe, das gewünschte Volumen an aufzuheizender Biomasse aufgeheizt.

Es wird noch darauf hingewiesen, daß die Zeichnungen lediglich schematische Beschreibungen einiger Ausführungsbeispiele zeigen und keine Maßstabszeichnungen darstellen. So kann z. B. in Figur 6 der Durchmesser des von der zylindrischen Heizungswand 22 begrenzten Hauptgärbereichs 68 etwa 5 m betragen, während der Durchmesser des relativ dick gezeichneten Zuführungsrohres 18 kleiner als 50 cm sein kann, so daß die Querschnittsfläche der Zuführungsleitung 18 nur ein kleiner Bruchteil der Querschnittsfläche des Hauptgärbehälters 68 ist, z. B. 1 : 80.

Bei dem Ausführungsbeispiel der Figur 6 ist die Göranlage in einem Endlager ausgebildet.

Weitere Änderungen und Ergänzungen der beschriebenen Ausführungsformen, zu welchen der Fachmann aufgrund des Studiums der obigen Beschreibung und ohne erfinderisches Zutun angeregt wird, fallen in den Schutzbereich der vorliegenden Erfindung. So ist zum Beispiel insbesondere bei freistehenden Hochbehältern die Einbringung der frischen Biomasse von unten in den Gärbehälter zweckmäßig. In diesem Fall muß, bei entsprechender Ausführungsform, die Biomasse nicht notwendigerweise auf die volle Behälterhöhe sondern lediglich auf die jeweilige Füllhöhe gepumpt werden.

Nach einer Weiterbildung der Erfindung ist die Folie als Haube ausgebildet, welche längs dem inneren Umfang des Gärbehälters auf dem vergärenden bzw. teilvergorenen Medium schwimmend gehalten wird. Die Folienhaube kann, wenn dieses Nachgärlager z. B. ringförmig ausgebildet ist, längs den Umfangsbereichen des gärenden bzw. nachgärenden Materials schwimmend gelagert sein.

Vorzugsweise tauchen der Rand bzw. die Ränder der Folienhaube in das vergärende bzw. nachvergärende Material ein, es kann aber auch eine Schürze längs den Rändern der Folienhaube vorgesehen sein, deren unterer Rand sich in dem gärenden bzw. nachgärenden Material befindet.

Verfahren und Vorrichtung
zur Erzeugung von Biogas

Ansprüche:

1.  Verfahren zur Erzeugung von Biogas aus organischen Materialien, insbesondere aus landwirtschaftlichen Reststoffen wie Gülle und Fäkalien, bei welchem die Biomasse in den unteren Bereich eines Gärbehälters eingebracht, im Durchgangsverfahren vergoren und nach Abgabe von Biogas nach unten abgeleitet wird, während das erzeugte Biogas oberhalb des Gärbehälters in einem Sammelbehälter aufgefangen und von dort agbeleitet wird,

dadurch gekennzeichnet,

daß die frische Biomasse in den unteren Bereich eines Teilraums des Gärbehälters eingeführt wird, dort auf einem Teil der Höhe dieses Teilraums von zwei Seiten aufgeheizt wird, wodurch der langsame Aufstieg der Biomasse unterstützt wird, und daß die Biomasse innerhalb des Gärbehälters absinkt und aus dem unteren Bereich des Gärbehälters durch Rohrleitungen abgeführt wird.

2.  Verfahren zur Erzeugung von Biogas aus organischen Materialien, insbesondere aus landwirtschaftlichen Reststoffen wie Gülle und Fäkalien, bei welchem die Biomasse in den unteren Bereich eines Gärbehälters eingebracht, vom Außenwandbereich her aufgeheizt, im Durchgangsverfahren vergoren und nach Abgabe von
Biogas nach unten abgeleitet wird, während das erzeugte
Biogas oberhalb des oder im Gärbehälter in einem
Sammelbehälter aufgefangen und von dort agbeleitet
wird, insbesondere nach Anspruch 1,
     dadurch gekennzeichnet,
daß die frische Biomasse in den unteren Bereich eines
äußeren Ringraums des Gärbehälters eingeführt wird,
dort auf einem Teil der Höhe zusätzlich von seinem
inneren Umfangsbereich her aufgeheizt wird, wodurch
der langsame Aufstieg der Biomasse unterstützt wird,
und daß die Biomasse im Inneren des Gärbehälters
absinkt und aus dem unteren Bereich des Gärbehälters
durch Rohrleitungen abgeführt wird.

3. Verfahren zur Erzeugung von Biogas aus organischen
Materialien, insbesondere aus landwirtschaftlichen
Reststoffen wie Gülle und Fäkalien, bei welchem die
Biomasse in den unteren Bereich eines Gärbehälters
eingebracht, im Durchgangsverfahren vergoren und
nach Abgabe von Biogas nach unten abgeleitet wird,
während das erzeugte Biogas oberhalb des oder im
Gärbehälter in einem Sammelbehälter aufgefangen und
von dort agbeleitet wird,
     dadurch gekennzeichnet,
daß die frische Biomasse in den unteren Bereich eines
inneren Ringraums des Gärbehälters eingeführt wird,
dort auf einem Teil der Höhe sowohl von dessen innerem
Umfangsbereich als auch zusätzlich vom äußeren Randbereich dieses inneren Ringraums her, der sich innerhalb
des Gärbehälters befindet, aufgeheizt wird, wodurch
der langsame Aufstieg der Biomasse unterstützt wird, und daß die Biomasse im äußeren Bereich innerhalb
des Gärbehälters absinkt und aus dem unteren Bereich
des Gärbehälters durch Rohrleitungen abgeführt wird.

4. Verfahren nach einem der Ansprüche 1 bis 3, dadurch
gekennzeichnet, daß die frische Biomasse durch die
vergärende bzw. vergorene Biomasse hindurch, dieser
Wärme entziehend und sich dadurch vorwärmend, eingeführt
wird.

5. Verfahren nach einem der Ansprüche 1 bis 4, dadurch
gekennzeichnet, daß die frische Biomasse im wesentlichen
im zentralen Bereich oder Umfeld des zentralen Bereichs
des Gärbehälters in diesen eingeführt und durch Verteilerrohre in Winkelbereiche im unteren Bereich des
Gärraums geleitet wird.

6. Verfahren nach einem der Ansprüche 1 bis 5, dadurch
gekennzeichnet, daß die nach unten gesunkene Biomasse
aus dem unteren Bereich des Gärbehälters seitlich,
nach oben oder durch den Boden des Gärbehälters ausgetragen wird.

7. Vorrichtung zur Erzeugung von Biogas aus Biomasse
insbesondere aus landwirtschaftlichen Reststoffen
wie Gülle und Fäkalien, im Durchgangsverfahren, mit
einer Zuführleitung für frische Biomasse, die in
den unteren Bereich eines Gärbehälters mündet, der
eine Heizvorrichtung umfaßt,
dadurch gekennzeichnet, daß die Zuführungsleitung (18) sich in den unteren Behälterraum erstreckt
und sich von dort in mindestens zwei Rohrleitungen (19, 19') verzweigt, die in einen Ringbereich (26)
des Gärbehälters münden, und daß Heizeinrichtungen
(12, 24, 72) auf zwei Seiten auf einem Teil dieses
Ringbereichs, die frisch eingeführte Biomasse von
zwei Seiten aufwärmend, vorgesehen sind.

8. Vorrichtung zur Erzeugung von Biogas aus Biomasse,
insbesondere aus landwirtschaftlichen Reststoffen
wie Gülle und Fäkalien, im Durchgangsverfahren, mit
einer Zuführleitung für frische Biomasse, die in
den unteren Bereich eines Gärbehälters mündet, der
im Bereich seiner Außenwand eine Heizvorrichtung
umfaßt,
    dadurch gekennzeichnet, daß die Zuführungsleitung (18) sich in den unteren Behälterraum erstreckt
und sich von dort in mindestens zwei Rohrleitungen
(19, 19') verzweigt, die in den äußeren Bereich des
Gärbehälters münden, und daß etwa im mittleren Abstand
zwischen Außenwand und Zentrum des Gärbehälters,
im wesentlichen in dessen unterem Abschnitt, eine
oder mehrere zusätzliche Heizeinrichtungen (24) vorgesehen sind.

9. Vorrichtung nach Anspruch 8, dadurch gekennzeichnet,
daß die zusätzliche Heizung (24) im oder am äußeren
Bereich der umlaufenden Wand (22) angeordnet und
eine Isolierung zur Hemmung des Wärmeübergangs von
der zusätzlichen Heizung zum inneren Bereich des
Gärbehälters vorgesehen ist.

10. Vorrichtung nach einem der Ansprüche 8 bis 9,
dadurch gekennzeichnet, daß die Gasaustragstelle

_ 41 _

11. Vorrichtung nach einem der Ansprüche 8 bis 10,
dadurch gekennzeichnet, daß eine Rührvorrichtung
vorgesehen ist, die Schwimmschichten (Leichtteile)-
und Sinkschichten (Sandteile, Acetat) die keinen
thermischen Auftrieb beinhalten, in Schwebezustand
versetzt werden und dadurch gezielt ausgetragen werden
können.

12. Vorrichtung zur Erzeugung von Biogas aus Biomasse,
insbesondere aus landwirtschaftlichen Reststoffen
wie Gülle und Fäkalien, im Durchgangsverfahren, mit
einer Zuführungsleitung für frische Biomasse, die
in den unteren Bereich eines Gärbehälters mündet,
der eine Heizvorrichtung umfaßt,
dadurch gekennzeichnet, daß die Zuführungsleitung (18) sich in den unteren Behälterraum erstreckt
und sich von dort in mindestens zwei Rohrleitungen
verzweigt, die in einen inneren Ringbereich (68)
des Gärbehälters münden, und daß Heizeinrichtungen
(24, 72) auf zwei Seiten auf einem Teil dieses Ringbereichs (68), die frisch eingeführte Biomasse von
zwei Seiten aufwärmend, vorgesehen sind.

13. Vorrichtung nach einem der Ansprüche 7 bis 12,
dadurch gekennzeichnet, daß die Zuführungsleitung
(18) aus wärmeleitendem Material besteht und sich
durch die vergärende bzw. vergorene Biomasse hindurch
erstreckt.

14. Vorrichtung nach Anspruch 13, dadurch gekennzeichnet,

- 42 -

daß die zusätzliche Heizung (24, 72) wärmespeicherndes
Material (22, 70) enthält.

15. Vorrichtung nach einem der Ansprüche 13 bis
14, dadurch gekennzeichnet, daß die zusätzliche Heizung
(24) im oder am äußeren Bereich der umlaufenden Wand
(22) angeordnet und eine Isolierung zur Hemmung des
Wärmeübergangs von der zusätzlichen Heizung zum äußeren
Bereich des Gärbehälters vorgesehen ist.

16. Vorrichtung nach einem der Ansprüche 13 bis
15, dadurch gekennzeichnet, daß auch die Zuführungsleitungen (18) von einem Heizmantel (70) umgeben
sind.

17. Vorrichtung nach einem der Ansprüch 13 bis 16,
dadurch gekennzeichnet, daß die Gasaustragstelle
als zentraler Gassammelring (16), Gassammelschacht
oder Gassack oder -haube ausgebildet ist.

18. Vorrichtung nach einem der Ansprüche 13 bis 17,
dadurch gekennzeichnet, daß eine Rührvorrichtung
vorgesehen ist, die Schwimmschichten (Leichtteile)-
und Sinkschichten (Sandteile, Acetat) die keinen
thermischen Auftrieb beinhalten, in Schwebezustand
versetzt werden und dadurch gezielt ausgetragen verden
können.

19. Vorrichtung nach einem der Ansprüche 7 bis 18,
dadurch gekennzeichnet, daß eine Gasauffanghaube
(82, 84) vorgesehen ist.

- 43 -

20. Vorrichtung nach Anspruch 19, dadurch gekennzeichnet, daß der Raum zwischen der Gasabdichthaube (82, 88) und der Oberseite (14) des Gärbehälters hinterlüftet ist.

21. Vorrichtung nach Anspruch 19 oder 20, dadurch gekennzeichnet, daß die zusätzliche Heizeinrichtung (22) als Teil einer Deckenstützeinrichtung ausgebildet ist.

22. Vorrichtung nach einem der Ansprüche 19 bis 21, dadurch gekennzeichnet, daß die Gäranlage in einem End- oder Nachgärlager ausgebildet ist.

23. Verfahren nach einem der Ansprüche 1 bis 10, dadurch gekennzeichnet, daß das erzeugte Biogas unter einer flexiblen Folienhaube aufgefangen wird, welche sich entsprechend der Höhe des Füllstandes der Biomasse und des Gases heben bzw. senken kann.

24. Vorrichtung nach einem der Ansprüche 11 bis 22, dadurch gekennzeichnet, daß die Folienhaube längs dem inneren Umfang des Gärbehälters auf dem Medium schwimmend gehalten wird.

25. Vorrichtung nach einem der Ansprüche 11 bis 22, dadurch gekennzeichnet, daß die Folienhaube längs den Umfangsbereichen des gärenden bzw. des nachgärenden Materials schwimmend gelagert ist.

26. Vorrichtung nach einem der Ansprüche 11 bis 22 oder 24 oder 25, dadurch gekennzeichnet, daß der

0158213

- 45 -

Rand bzw. die Ränder der Folienhaube in das vergärende bzw. nachvergärende Material eintaucht.

27. Vorrichtung nach einem der Ansprüche 11 bis 26, dadurch gekennzeichnet, daß längs dem bzw. den Rändern der Folienhaube eine Schürze vorgesehen ist, deren unterer Rand sich in dem gärenden bzw. nachgärenden Material befindet.

FIG. 1

0158213

FIG. 2

0158213

FIG. 3

FIG. 4

FIG. 5

0158213

# FIG. 6